# EUROPEAN PATENT SPECIFICATION

(11) **EP 3 278 778 B1**
(45) Date of publication and mention of the grant of the patent: **29.03.2023**
(21) Application number: 16183095.5
(22) Date of filing: 05.08.2016
(51) Int. Cl.: A61F 13/15, B05C 11/02, B05C 19/04, A61F 13/53

(54) **IMPROVED PARTICLE DISPERSING DEVICE AND METHOD FOR ABSORBENT ARTICLES**
VERBESSERTE PARTIKELDISPERGIERVORRICHTUNG UND -VERFAHREN FÜR ABSORBIERENDE ARTIKEL
DISPOSITIF DE DISPERSION DE PARTICULES AMÉLIORÉ ET PROCÉDÉ POUR ARTICLES ABSORBANTS

(43) Date of publication of application: 07.02.2018
(73) Proprietor: Ontex BV, 9255 Buggenhout (BE)
(72) Inventor: HEEGE, Thomas, 56727 Mayen (DE); WEBER, Ainas, 56727 Mayen (DE)
(74) Representative: Saurat, Thibault

(56) References cited:
- EP-A1- 0 613 726
- EP-A1- 2 777 664
- WO-A1-2012/090508
- US-A- 4 927 582

## Description

### TECHNICAL FIELD

The invention pertains to the technical field of absorbent articles which can be used to manufacture absorbent bodies for use in absorbent hygienic disposables such as disposable diapers and the like and a manufacturing method therefor. In particular, the invention concerns methods and apparatuses for distributing absorbent material comprising absorbent particles onto a moving substrate, preferably a nonwoven.

### BACKGROUND

Hygienic disposables such as baby diapers, incontinence diapers, diaper pants, panty liners, sanitary napkins, etc., need to be absorbent to bodily exudates. In order to do so, the disposables typically comprise an absorbent core or body which is capable of quickly absorbing and retaining the exudates.

An absorbent core which is used in absorbent hygienic disposables typically comprises a layered structure with at least one carrier layer, typically a nonwoven, which is provided with absorbent material. This absorbent material may comprise fluff, which can be natural or synthetic or a mixture thereof. Cellulose fluff is commonly used. The absorbent material may comprise, alternative or in addition to fluff, absorbent particles. Superabsorbent polymer (SAP) particles are commonly used. The absorbent core may further also comprise a covering layer, e.g. a covering nonwoven, such that the absorbent core basically consists of a sandwich structure wherein the absorbent material is sandwiched between a carrier and a covering layer.

In the past, a number of methods and apparatuses have been developed to provide a carrier with absorbent material. However, these methods and apparatuses seem to lack flexibility with respect to adapting how and which absorbent material is distributed on the carrier.

There remains a need in the art for a more efficient and flexible way to produce absorbent articles and cores comprising absorbent particles, in particular SAP particles. More particularly, the methods and apparatuses according to the state of the art lack an integrated way to distribute SAP on a non-woven web in such a manner that an optimal particle distribution profile can be obtained. Also, the methods and apparatuses according to the state of the art lack flexibility in changing the distribution and nature of the absorbent material on the carrier.

The method and apparatus disclosed in document EP1366825 utilizes the Venturi effect to suck the stream of SAP into the dispersion chamber. By modulation of the auxiliary stream these systems can create a pattern on the web. This approach however is rather unresponsive and very sensitive to speed alterations of the web below causing a lot of errors during the production process.

The method and apparatus according to EP2671549 oversaturates the 3D profile of the drum with SAP and then removes the surplus of SAP with a brush like device. The removed SAP has then either to be discarded or recirculated into the process. The approach therefore either creates lots of trash or requires a rather complex recirculation subsystem to make it work efficiently.

Another approach disclosed in document US4800102 utilizes a rotating disc with different openings as a valve of an antechamber to apply the SAP to the web in a controlled fashion. Since the stream of SAP to the antechamber has to be higher compared to the amount of SAP leaving the antechamber with each cycle there is always a surplus of SAP accumulating in the antechamber. This surplus again requires a rather complex recirculation subsystem or has to be discarded.

Document WO2010103453 discloses a method and apparatus involving a valve means by which the SAP can be dispersed intermittently and controllably. Similarly as in EP1366825 the modulation of the auxiliary stream enables creating a pattern on the web, yet suffers the same drawbacks as mentioned above for EP1366825.

Document EP0613726 discloses a process and apparatus involving an elastic pipe connected to a driving wheel, and suffer the same drawbacks as mentioned herein.

The present invention aims to resolve at least some of the problems mentioned above.

The invention thereto aims to provide methods and apparatuses which provide a flexible way for providing a carrier, preferably a nonwoven carrier, with absorbent material according to a distribution profile, wherein the distribution profile and/or the nature of the absorbent material can be easily adapted according to specific needs or in order to search for the optimal distribution.

The method and apparatus of the present invention offers a way to distribute absorbent particles, in particular SAP particles, that is responsive, can adapt to changes in the production process in a very short time, does not require any recirculation of the SAP and does not produce any relevant amounts of trash.

### SUMMARY OF THE INVENTION

The present invention provides a method and apparatus according to the subject-matter of claims 1 and 4, respectively, for distributing absorbent particles onto an endless moving substrate, preferably a nonwoven, and an absorbent article comprising a layer, preferably a nonwoven layer, provided with absorbent particles according to a distribution profile. The present invention also provides an apparatus and method according to claims 9 and 12, respectively, for providing absorbent material comprising fluff and absorbent particles onto an endless moving substrate, preferably a nonwoven.

In a first aspect, the present invention provides a method for distributing absorbent particles onto an endless moving substrate, preferably a nonwoven, which is being conveyed along a machine direction through a particle depositing chamber, by deflecting incoming absorbent particles by an oscillating impact means within said particle depositing chamber before deposition, thereby distributing the particles onto the moving substrate according to a distribution profile.

The terms "nonwoven", "nonwoven fabric", "web" or "nonwoven web" are used interchangeably and refer to a sheet material having a structure of individual fibers or threads which are interlaid, but not in a regular manner such as occurs with knitting or weaving processes. Nonwoven fabrics or webs can be formed from many processes such as for example, meltblowing processes, spunbonding processes, and bonded carded web processes.

The term "endless substrate" as used herein is known in the art and essentially refers to a substrate in the form of a two-dimensional sheet, wherein the substrate is limited in one dimension, i.c. the width-wise, transverse or cross direction, but basically is unlimited in the other dimension, i.c. the length-wise, longitudinal or machine direction. An "endless substrate" is an elongated strip of flat material essentially without beginning or end along the longitudinal direction. The machine direction is the direction along which the substrate moves, equal to said longitudinal direction. The cross direction is a direction parallel to the substrate and perpendicular to the machine direction. In practice, an endless substrate can typically be provided by a sheet material on a roll, which is unwound, thereby providing the endless substrate, the width of the substrate being determined by the width of the roll and the length of the substrate being determined by the length of the substrate which can be wound onto the roll, which can be much larger than the width and can essentially be deemed endless. Preferably, the substrate of the present invention is a nonwoven web.

The term "absorbent particles" refer to particulate material capable of absorbing liquids, in particular bodily exudates and which are used in absorbent hygienic disposable. Preferably, the absorbent particles are, or comprise, "Superabsorbent polymer particles" or "SAPs" which refer to water-swellable, water-insoluble organic or inorganic materials capable, under the most favorable conditions, of absorbing at least about 10 times their weight, or at least about 15 times their weight, or at least about 25 times, suitably about 30 times, and possibly about 60 times or more their weight in an aqueous solution containing 0.9 weight percent sodium chloride. In absorbent articles, such as diapers, incontinent diapers, baby diapers, diaper pants, sanitary napkins, panty liners, etc., the particle size is typically ranging between 100 to 800 µm, preferably between 300 to 600 µm, more preferably between 400 to 500 µm.

The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof. The hydrogel-forming polymers may be lightly crosslinked to render the material substantially water insoluble. Crosslinking may, for example, be by irradiation or covalent, ionic, Van der Waals, or hydrogen bonding. The superabsorbent material may be included in the absorbent layer or other fluid storage layer of an absorbent hygienic article of the present invention in an amount up to about 60% by weight. Typically, the superabsorbent material, when present, will be included in an amount of about 5% to about 40% by weight, based on the total weight of the absorbent core. However, more than 40% by weight can also be included.

The endless substrate is passed through a particle depositing chamber. A particle deposition chamber refers to an enclosed environment where the particles can enter through a particle inlet and can subsequently be deposited on the substrate. Hereby, the enclosed environment ensures that the bulk of the particulate material effectively is deposited on the substrate and/or that particulate material which is not deposited on the substrate can be controllably evacuated from the chamber. Typically, a particle depositing chamber comprises a set of upstanding side walls and a bottom which can be defined by the passing substrate. The upstanding side walls hereby delimit the sideways movements of the particles during deposition and essentially ensure that a particle introduced in the chamber is deposited on the passing substrate which defines the bottom of the chamber. The upstanding sidewalls hereby can preferably have two oppositely disposed openings for allowing the substrate the pass into and out of the enclosure defined by the side walls. Typically, the openings are thin slits. The bottom of the chamber can be defined by the substrate, i.e. the substrate can essentially close off the depositing chamber at the bottom for the particles. The substrate can also be transported over a bottom plate or by using an endless conveying means such as a rotating drum, in which case the bottom of the chamber can also be at least partially defined by said bottom plate or said conveying means. The chamber may be open from above. Preferably, however, the chamber is at least partially closed at the top, e.g. by a lid or a ventilation system.

Absorbent particles are introduced into the particle depositing chamber, preferably via an inlet disposed above the substrate. Before being deposited on the substrate, at least a portion of the absorbent particles are deflected by a moving, and in particular an oscillating impact means. The portion which is deflected is preferably a substantial portion, e.g. at least 50% , more preferably at least 60%, yet more preferably at least 70%, still more preferably at least 80% or more, such as 81%, 82%, 83%, 84%, 85%, 86%, 87%, 88%, 89%, 90%, 91%, 92%, 93%, 94%, 95%, 96%, 97%, 98%, 99%, 100%, or any value therebetween.

By deflecting the absorbent particles prior to deposition, the inventors have found that two effects occur, each of which increases the flexibility and ease of the distribution process.

Firstly, a distribution profile of absorbent particles deposited on the substrate is obtained. Hereby, the term "distribution profile" refers to a non-homogeneous distribution of the particle density per unit of surface area of the substrate. Because of the oscillating movement of the impact means, the distribution profile of particles on the conveyed substrate is essentially periodical. When the absorbent article is, in a subsequent step, cut in order to manufacture an absorbent core for a hygienic disposable, the periodical distribution profile allows the absorbent cores to be essentially the same with respect to the distribution of the absorbent particles.

Secondly, by deflecting the particles with a moving impact means, the inventors have observed that certain undesired systematically occurring problems can be avoided.

One such problem is "caking": when SAP particles accumulate in certain places within an absorbent core, they can fuse or bake together during use and thereby reduce the amount of liquids that can be stored, as well as reduce the comfort of wearing a diaper due to an uneven feel. Caking may be caused by an uneven distribution of the SAP particles during production or by freely wandering SAP particles inside the core. The inventors have found that at least the former cause is substantially decreased by using oscillating impact means to deflect the absorbent particles. Without wishing to be bound by theory, it is assumed that by deflecting an absorbent particle's trajectory with a moving, i.c. oscillating, impact means within the depositing chamber, a randomization occurs: two particles arriving within a short but finite period at the same position of the impact means will be deflected differently because of the small but finite change in position, rotational speed and direction of the impact means.

The use of an oscillating impact means further allows easy control on the distribution profile of the deposited particles by altering the oscillating movement of the impact means. Whereas prior art methods and apparatuses may require substantial hardware modifications of the distribution means in order to change the distribution profile deposited on the substrate, the present invention can change the profile much more easily, e.g. by a software-like modification of the actuation of the impact means whereby the rotational speed profile can be adapted as desired, or by a simple hardware modification such as a change in transmission between an actuator and a pivot axis of the impact means.

In a related aspect, the present invention concerns an apparatus for distributing absorbent particles onto an endless substrate, the apparatus comprising a particle depositing chamber comprising one or more openings for conveying the substrate along a machine direction through the particle depositing chamber, said particle depositing chamber comprising a particle inlet for introducing absorbent particles into the particle depositing chamber, whereby the apparatus comprises an impact means which is pivotably mounted within the particle depositing chamber, the impact means being configured to oscillate for deflecting absorbent particles coming in through the particle inlet before deposition of the particles onto the substrate according to a distribution profile.

The apparatuses and methods according to the present invention allow production of an absorbent article comprising absorbent particles which are distributed according to a distribution profile. Hence, the present disclosure also concerns an absorbent article comprising a substrate layer provided with absorbent particles according to a distribution profile, said distribution profile comprising a length-wise profile of the distribution which is periodical with a period length, whereby in a single period, and preferably for each period, said length-wise profile comprises:
- at least a minimal, non-zero particle density over essentially the full period length;
- a maximum particle density at a first length-wise position, and
an insult region spanning at most a 50% portion of the period length and comprising at least 60% of the absorbent particles distributed on said absorbent article over the period length.

In a second aspect, the present invention concerns an apparatus for providing absorbent material comprising fluff and absorbent particles onto an endless substrate, said apparatus comprising:
- an endless carrier means for conveying said endless substrate in a machine direction;
- a particle depositing chamber comprising one or more openings for conveying the substrate along a machine direction through the particle depositing chamber, said particle depositing chamber comprising a particle inlet for introducing absorbent particles or a mixture of absorbent particles and fluff into the particle depositing chamber;
wherein the apparatus comprises a fluff depositing chamber comprising one or more openings for conveying the substrate along a machine direction through the fluff depositing chamber, the fluff deposition chamber comprising an inlet for fluff, wherein said fluff depositing chamber is disposed before or after said particle depositing chamber along the machine direction. In a particularly preferred embodiment, the particle depositing chamber comprises mounting means for pivotably mounting impact means within said particle depositing chamber. Hence, in a similar aspect, the present invention concerns a method for providing absorbent material comprising fluff and absorbent particles onto an endless substrate with an apparatus comprising an endless carrier means, a particle depositing chamber, a fluff depositing chamber and optionally an additional fluff depositing chamber, the method comprising the steps of:
(a) conveying an endless substrate on an endless carrier means along a machine direction through a fluff depositing chamber and subsequently through a particle depositing chamber and optionally subsequently through an additional fluff depositing chamber;
(b) optionally depositing fluff on said substrate in said first depositing chamber;
(c) depositing absorbent particles on said substrate in said second depositing chamber, optionally said absorbent particles being mixed with fluff;
(d) optionally depositing fluff on said substrate in said third depositing chamber.

The above apparatus allows to provide the substrate with a combination of absorbent materials, preferably a combination of fluff and absorbent particles. Furthermore, the apparatus is versatile with respect to the nature and amount of absorbent material which is provided on the substrate. Hereby it is possible to provide the substrate with:
- fluff only, e.g. by shutting down the supply of absorbent particles to the particle depositing chamber;
- absorbent particles only, e.g. by shutting down the supply of fluff to the fluff depositing chamber;
- a first layer of fluff and a second layer of absorbent particles in the case the fluff deposition chamber is disposed before the particle depositing chamber;
- a first layer of absorbent particles and a second layer of fluff in the case the fluff deposition chamber is disposed after the particle depositing chamber;
- a first layer of fluff and a second layer of a mixture of fluff and absorbent particles in the case the fluff deposition chamber is disposed before the particle depositing chamber, e.g. by providing the particle depositing chamber with a mixture of fluff and absorbent particles;
- a first layer of a mixture of fluff and absorbent particles and a second layer of fluff in the case the fluff deposition chamber is disposed after the particle depositing chamber, e.g. by providing the particle depositing chamber with a mixture of fluff and absorbent particles;
- a first layer of fluff, a second layer of absorbent particles or a mixture of absorbent particles and fluff and a third layer of fluff in case the fluff depositing chamber is disposed before the particle depositing chamber and an additional fluff depositing chamber is disposed after the particle depositing chamber,
and this without any modifications to the apparatus or production line as such.

In an embodiment, the fluff depositing chamber and the particle depositing chamber are disposed next to each other and may share a common wall with an opening for direct passage of the substrate from one depositing chamber to the other. If an additional fluff depositing chamber is provided, it is preferably provided next to the particle depositing chamber and may share a common wall with the particle depositing chamber with an opening for direct passage of the substrate from one depositing chamber to the other.

In case mounting means are provided in the particle depositing chamber for pivotably mounting impact means within the particle depositing chamber, an impact means can be easily mounted into that chamber. Preferably, such an impact means is pivotably mounted into the particle depositing chamber for distributing the absorbent particles according to a distribution profile.

### DESCRIPTION OF FIGURES

**Figure 1** shows an embodiment of an apparatus for distributing SAP and optionally fluff according to the present invention from a perspective view.
**Figure 2** shows an embodiment of an apparatus for distributing SAP and optionally fluff according to the present invention in a side view.
**Figure 3** shows an example of an impact means according to the present invention.
**Figure 4** shows an example of a distribution profile that can be produced by an apparatus for distributing SAP according to the present invention.
**Figures 5 to 7** illustrate an embodiment of a method for distributing absorbent particles onto a substrate according to the present invention, using an embodiment of an apparatus according to the present invention.

### DETAILED DESCRIPTION OF THE INVENTION

The present invention concerns a method and apparatus for distributing absorbent particles onto an endless substrate, as disclosed in the claims and further in the present document. The present invention also concerns an apparatus and method for providing absorbent material comprising fluff and absorbent particles onto an endless substrate, as well as the absorbent article comprising a substrate layer provided with absorbent material comprising fluff and absorbent particles as disclosed in the claims and further in this document.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
The terms "absorbent hygienic article", "hygienic article", "hygienic disposable" and "disposable hygienic article" as used herein, refer to devices that absorb and contain liquid, and more specifically, refer to devices that are placed against or in proximity to the body of the wearer to absorb and contain the various exudates discharged from the body. Hygienic articles include but are not limited to diapers, adult incontinence briefs, training pants, diaper holders and liners, sanitary napkins and the like, as well as surgical bandages and sponges. Hygienic articles preferably comprise a longitudinal axis and a transversal axis perpendicular to said longitudinal axis. The longitudinal axis is hereby conventionally chosen in the front-to-back direction of the hygienic article when referring to the hygienic article being worn, and the transversal axis is conventionally chosen in the left-to-right direction of the hygienic article when referring to the hygienic article being worn. Disposable hygienic articles can include a liquid pervious top sheet, a back sheet joined to the top sheet, and an absorbent core positioned and held between the top sheet and the back sheet. The top sheet is operatively permeable to the liquids that are intended to be held or stored by the hygienic article, and the back sheet may or may not be substantially impervious or otherwise operatively impermeable to the intended liquids. The hygienic article may also include other components, such as liquid wicking layers, liquid intake layers, liquid distribution layers, transfer layers, barrier layers, wrapping layers and the like, as well as combinations thereof. Disposable hygienic articles and the components thereof can operate to provide a body-facing surface and a garment-facing surface.

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "% by weight" (weight percent), here and throughout the description unless otherwise defined, refers to the relative weight of the respective component based on the overall weight of the formulation.

The term "insult", here and throughout the description, refers to an exposure to an amount of bodily exudates in a limited region and during a limited period. Typically, exposure of a diaper to bodily exudates can occur in the form of insults, such as 100 ml of urine is deposited in the center of the crotch region of a diaper within about 10 seconds. Hence, the term "insult region" refers to the region of an absorbent article where insults occur or are expected to occur.

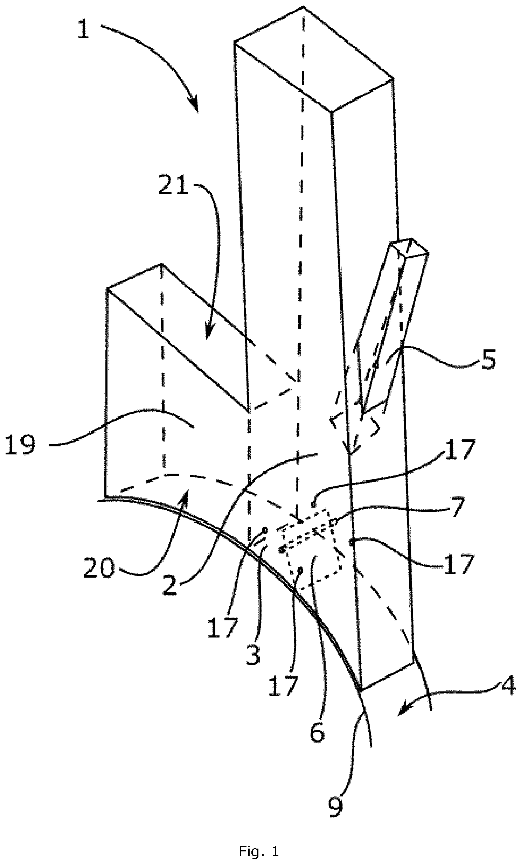

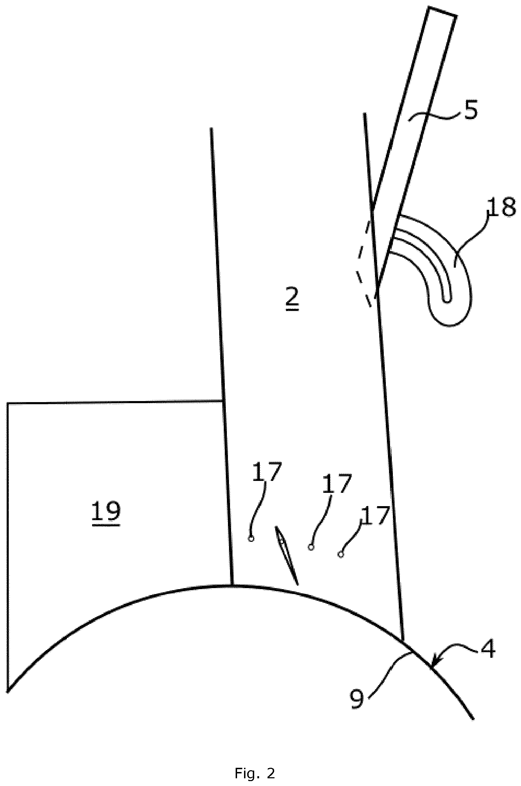

Figures 1 and 2 show an embodiment of an apparatus (1) for distributing SAP and optionally fluff according to the present invention from a perspective view. The apparatus (1) comprises a particle depositing chamber (2) which comprises an opening (3) at the bottom side for conveying the substrate (4) along a machine direction through the particle depositing chamber, i.e. in the shown embodiment, the substrate defines the bottom of the particle depositing chamber. The particle depositing chamber (2) comprises a particle inlet (5) for introducing absorbent particles into the particle depositing chamber (2). The apparatus further comprises an impact means (6) in the form of a plate, which is pivotably mounted within the particle depositing chamber (2) around a pivot axis (7). The impact means are configured to oscillate for deflecting absorbent particles coming in through the particle inlet (5) before deposition of the particles onto the substrate (4) according to a distribution profile.

The apparatus may comprise an inlet orientation system (18) which allows to change the direction at which the particles are introduced into the chamber by changing the angle of the inlet (5).

In a preferred embodiment of the invention, the absorbent particles are superabsorbent polymer (SAP) particles (11).

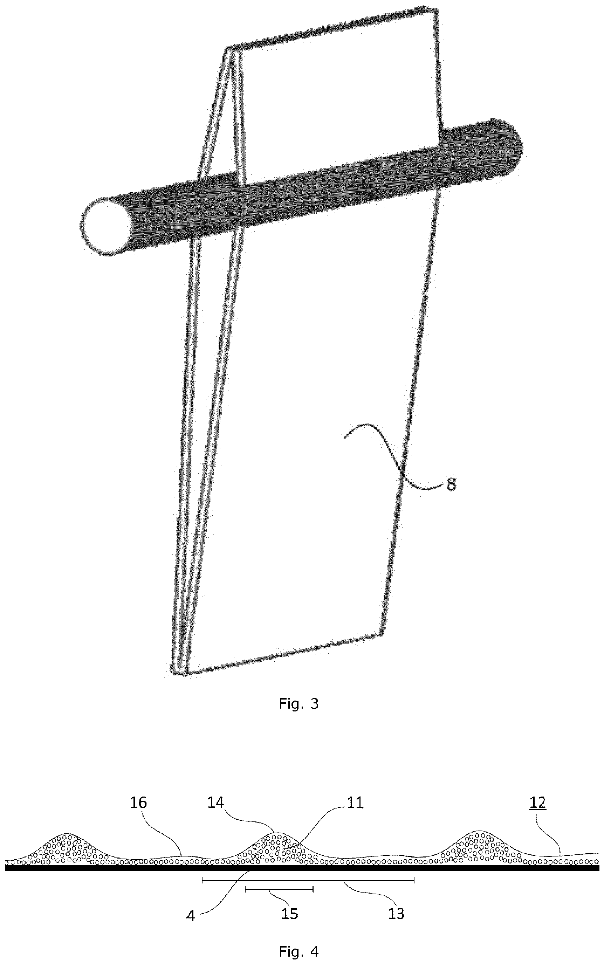

According to the invention, the said impact means comprises a rigid plate (10), preferably a wedge-shaped rigid plate (8), as illustrated in fig. 3.

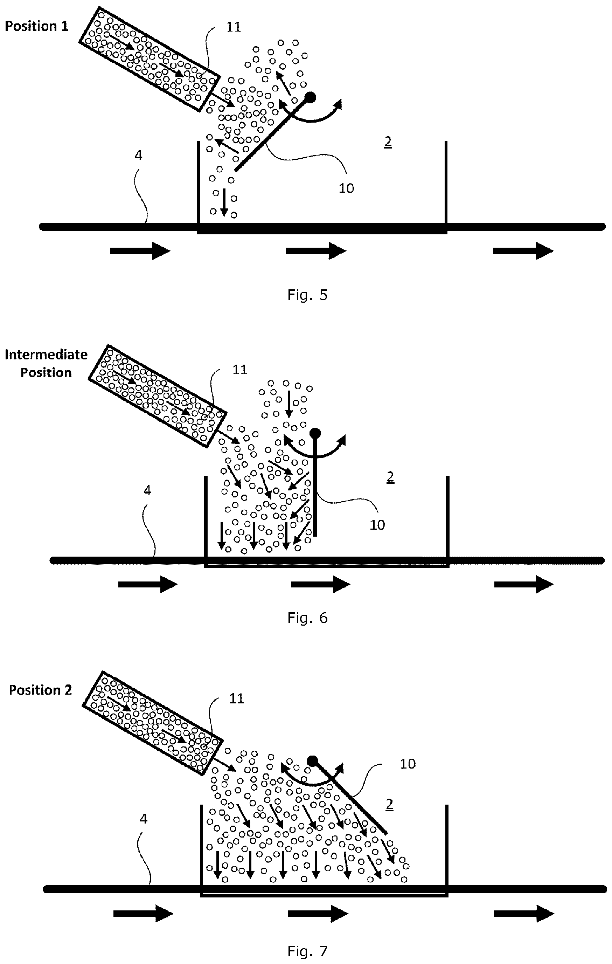

In an embodiment, the machine direction is a substantially horizontal direction at the location of the particle depositing chamber, as illustrated in figs. 5 to 7. However, the machine direction may also be slanted or curved, hereby following e.g. the curvature of a rotating drum (9) which can be used as an endless conveying means as illustrated in figs. 1 and 2.

In an embodiment of the method according to the present invention, the impact means (6,8,10) oscillates around a pivot axis (7) oriented along an essentially horizontal cross direction, preferably above the substrate (4). In an embodiment of the apparatus, the impact means (6,8,10) is pivotable around a pivot axis (7) oriented along an essentially horizontal cross direction, preferably above the one or more openings for conveying the substrate (4).

According to the invention, the impact means oscillates according to a pre-defined oscillation profile such that the absorbent particles are distributed onto the substrate according to a distribution profile as illustrated in fig. 4, resulting in an absorbent article (16). The distribution profile (12) is essentially periodical whereby in a single period (13), and preferably for each period, the length-wise profile comprises:
- at least a minimal, non-zero particle density over essentially the full period length;
- a maximum particle density at a first length-wise position (14), and
- an insult region (15) spanning at most a 50% portion of the period length and comprising at least 60% of the absorbent particles distributed on said absorbent article (16) over the period length.

In an embodiment, the impact means is removably mounted in the particle depositing chamber. This allows easy replacement and/or repositioning of the impact means. In figs. 1 and 2, the particle depositing chamber is provided with a number of mounting means (17) for pivotable mounting the impact means. These mounting means can take the form of holes in opposite side walls of the chamber through which the pivot axis of the mounting means can be inserted. The unused holes can be closed off. In this embodiment, the impact means can be actuated via the pivot axis which sticks out of the particle depositing chamber.

In an embodiment, the apparatus comprises an endless carrier means, preferably a rotating drum (9), for conveying said endless substrate in the machine direction.

In a preferred embodiment, the apparatus comprises a fluff depositing chamber (19) comprising one or more openings (20) for conveying the substrate (4) along a machine direction through the fluff depositing chamber (19), said fluff depositing chamber (19) disposed before or after said particle depositing chamber (2) along the machine direction, preferably before said particle depositing chamber (2) along the machine direction as illustrated in figs. 1 and 2, the fluff deposition chamber (19) comprising an inlet for fluff (21) or for a mixture of fluff and absorbent particles, and optionally comprising an inlet for absorbent particles.

The utility of a distribution chamber for the distribution method lies in the confinement of absorbent particles within the physical space of the chamber. Regardless whether being impacted by the impact means or not, absorbent particles are preferably prevented from falling next to the non-woven web. This can realized by two or more machine-directed walls of the chamber, oriented along the machine direction. Additionally, regardless whether being impacted by the impact means or not, in order to obtain a distribution of absorbent particles in a controllable/controlled fashion, the particles preferably do not fall beyond the confines of a certain region of said web, said region identifying at each moment the region of the web onto which the particles are currently being distributed. This can be realized by two or more cross-directed walls of the chamber along the cross direction. Furthermore, the distribution chamber may comprise a bottom plate above which the non-woven web is moving in the machine direction, in which case the two or more cross-directed walls comprise slits along the cross direction in their lower portions, representing a web inlet and a web outlet, respectively. In the alternative and preferred case where the distribution chamber does not comprise a bottom plate or in the absence of a distribution chamber, said web may be attached to an endless conveying means such as a rotating drum that causes the web to move along the machine direction. Preferably the attachment is done by vacuum suction means.

An embodiment of the method and apparatus according to the present invention are exemplified in figs. 5 to 6. In this embodiment, a movable plate (10) inside a particle depositing chamber (2) is made to oscillate while a stream of SAP particles (11) is introduced into the chamber (2) under an angle.

The plate (10) shifts back and forth between position 1 (fig. 5) and position 2 (fig. 7) like a pendulum. The SAP particles (11) are blown into the depositing chamber (2) through for example a feeding pipe, a nozzle or by any other means. Depending on the current angle of the plate, some of the SAP particles will either hit the plate or descend directly onto the substrate (4) that moves through the depositing chamber.

When a particle hits the plate under an angle, it will bounce of its surface in a deflection angle which is almost a mirror-reflection angle. Note that due to movement of the plate, the deflection angle can be different from a mirror-reflection angle.

In the shown embodiment, when particles can bounce of the plate in an upward direction, hereby creating a cloud of particles that will descend with some delay to the web once the plate has moved out of the way (intermediate position in fig. 6 and position 2 in fig. 7). This delay allows the system to create an asymmetrical pattern on the web (see e.g. fig. 4), with longer spans of less SAP and shorter spans of more SAP.

The effect can be increased by altering the way the plate is moved. When reaching its final position on one side, the plate can stay there for a longer duration, thereby effectively blocking off the way of the SAP (position 1) or likewise opening up the way (position 2). In block-off position (position 1) the SAP will accumulate into a rather dense cloud above the plate. Some of the SAP will immediately fall onto the plate letting it also act similar to a trapdoor the moment the plate will start to move towards the second position.

Alternative or additional to using the gravitational pull to cause the downward movement of the SAP, the depositing chamber (2) can also be built as a vacuum chamber creating a pull on the absorbent particles via a difference in pressure level, i.e. by a pressure gradient. Since the vacuum will also cause the web to be tightly pressed onto the bottom of the depositing chamber, the bottom plate can be part of a rotating drum and not fixed to the particle depositing chamber. To create additional patterns of absorbent particles on the web, the drum may comprise a 3D profile on its outer surface that the web is tightly pressed into.

In an embodiment, the absorbent particles are provided into the particle depositing chamber in a metered flow.

In an embodiment, the particle inlet comprises a rectangular cross section, preferably a slit-like cross section.

## Claims

1. Method for distributing absorbent particles onto an endless substrate, preferably a nonwoven, which is being conveyed along a machine direction through a particle depositing chamber, by deflecting incoming absorbent particles by an oscillating impact means within said particle depositing chamber before deposition, thereby distributing the particles onto the substrate according to a distribution profile, wherein said impact means comprises a rigid plate (10), which is pivotably mounted within the particle depositing chamber around a pivot axis,
wherein said impact means oscillates according to a pre-defined oscillation profile such that the absorbent particles are distributed onto the substrate according to a distribution profile comprising an essentially periodical length-wise profile whereby in a single period, and preferably for each period, said length-wise profile comprises:
∘ at least a minimal, non-zero particle density over essentially the full period length;
o a maximum particle density at a first length-wise position, and
∘ an insult region spanning at most a 50% portion of the period length and comprising at least 60% of the absorbent particles distributed on said substrate over the period length.

2. Method according to claim 1 wherein the particles are superabsorbent polymer particles and/or wherein the substrate is a nonwoven.

3. Method according to claim 1 or 2 wherein said impact means oscillates around a pivot axis above the substrate, the pivot axis being oriented along an essentially horizontal cross direction.

4. Apparatus for distributing absorbent particles onto an endless substrate and optionally fluff for use in a method according to any of the preceding Claims, the apparatus comprising a particle depositing chamber comprising one or more openings for conveying the substrate along a machine direction through the particle depositing chamber, said particle depositing chamber comprising a particle inlet for introducing absorbent particles into the particle depositing chamber,
**characterized in that** the apparatus comprises an impact means, wherein said impact means comprises a rigid plate (10), which is pivotably mounted within the particle depositing chamber, the impact means being configured to oscillate for deflecting absorbent particles coming in through the particle inlet before deposition of the particles onto the substrate according to a distribution profile, said distribution profile comprising an essentially periodical length-wise profile whereby in a single period, and preferably for each period, said length-wise profile comprises:
∘ at least a minimal, non-zero particle density over essentially the full period length;
∘ a maximum particle density at a first length-wise position, and
∘ an insult region spanning at most a 50% portion of the period length and comprising at least 60% of the absorbent particles distributed on said substrate over the period length.

5. Apparatus according to claim 4 wherein said impact means comprises a wedge-shaped rigid plate.

6. Apparatus according to claim 4 or 5 wherein the impact means is removably mounted in the particle depositing chamber.

7. Apparatus according to any of the claims 4 to 6, comprising an endless carrier means, preferably a rotating drum, for conveying said endless substrate in the machine direction.

8. Apparatus according to any of the claims 4 to 7, comprising a fluff depositing chamber comprising one or more openings for conveying the substrate along a machine direction through the fluff depositing chamber, said fluff depositing chamber disposed before or after said particle depositing chamber along the machine direction, preferably before said particle depositing chamber along the machine direction, the fluff deposition chamber comprising an inlet for fluff or for a mixture of fluff and absorbent particles, and optionally comprising an inlet for absorbent particles.

9. Apparatus for providing absorbent material comprising fluff and absorbent particles onto an endless substrate, said apparatus comprising:
o an endless carrier means for conveying said endless substrate in a machine direction;
∘ an apparatus for distributing absorbent particles according to claim 4 to 8, said particle depositing chamber comprising a particle inlet for introducing absorbent particles or a mixture of absorbent particles and fluff into the particle depositing chamber;
**characterized in that** the apparatus comprises a fluff depositing chamber comprising one or more openings for conveying the substrate along a machine direction through the fluff depositing chamber, the fluff deposition chamber comprising an inlet for fluff, wherein said fluff depositing chamber is disposed before or after said particle depositing chamber along the machine direction,
and **in that** the particle depositing chamber comprises mounting means for pivotably mounting impact means within said particle depositing chamber.

10. Apparatus according to claim 9 wherein said fluff depositing chamber is disposed before said particle depositing chamber along the machine direction.

11. Apparatus according to any of claims 9 or 10, comprising an additional fluff depositing chamber comprising one or more openings for conveying the substrate along a machine direction through the additional fluff depositing chamber, the additional fluff deposition chamber comprising an inlet for fluff wherein said additional fluff depositing chamber is disposed after said particle depositing chamber along the machine direction.

12. Method, for providing absorbent material comprising fluff and absorbent particles onto an endless substrate with an apparatus comprising an endless carrier means, a particle depositing chamber, a fluff depositing chamber and optionally an additional fluff depositing chamber, the method comprising the steps of:
(a) conveying an endless substrate, preferably a nonwoven, on an endless carrier means along a machine direction through a fluff depositing chamber and subsequently through a particle depositing chamber and optionally subsequently through an additional fluff depositing chamber;
(b) optionally depositing fluff on said substrate in said first depositing chamber;
(c) depositing absorbent particles on said substrate in said second depositing chamber, optionally said absorbent particles being mixed with fluff;
(d) optionally depositing fluff on said substrate in said third depositing chamber,
said method being performed using an apparatus according to any of the claims 9 to 11.

## Patentansprüche

1. Verfahren zur Verteilung von absorbierenden Teilchen auf einem Endlossubstrat, vorzugsweise einem Vlies, das entlang einer Maschinenrichtung durch eine Teilchenabscheidekammer befördert wird, indem eingehende absorbierende Teilchen durch ein schwingendes Stoßmittel innerhalb der Teilchenabscheidekammer vor ihrer Abscheidung abgelenkt werden, um dadurch die Teilchen auf dem Substrat gemäß einem Verteilungsprofil zu verteilen, wobei das Stoßmittel eine starre Platte (10) umfasst, die innerhalb der Teilchenabscheidekammer um eine Drehachse drehbar befestigt ist,
wobei das Stoßmittel gemäß einem vordefinierten Schwingungsprofil schwingt, derart dass die absorbierenden Teilchen auf dem Substrat gemäß einem Verteilungsprofil verteilt werden, das ein im Wesentlichen periodisches längsgerichtetes Profil umfasst, wobei das längsgerichtete Profil in einer einzelnen Periode und vorzugsweise für jede Periode umfasst:
o mindestens eine minimale, von null verschiedene Teilchendichte über im Wesentlichen die vollständige Periodenlänge;
o eine maximale Teilchendichte in einer ersten längsgerichteten Position, und
o eine Insultregion, die sich höchstens über einen 50%igen Teil der Periodenlänge erstreckt und mindestens 60% der absorbierenden Teilchen umfasst, die auf dem Substrat über die Periodenlänge verteilt sind.

2. Verfahren nach Anspruch 1, wobei die Teilchen superabsorbierende Polymerteilchen sind, und/oder wobei das Substrat ein Vlies ist.

3. Verfahren nach Anspruch 1 oder 2, wobei das Stoßmittel um eine Drehachse über dem Substrat schwingt, wobei die Drehachse entlang einer im Wesentlichen horizontalen Querrichtung orientiert ist.

4. Vorrichtung zum Verteilen von absorbierenden Teilchen auf einem Endlossubstrat und optional Flusen zur Verwendung in einem Verfahren nach einem der vorhergehenden Ansprüche, wobei die Vorrichtung eine Teilchenabscheidekammer mit einer oder mehreren Öffnungen zum Befördern des Substrats entlang einer Maschinenrichtung durch die Teilchenabscheidekammer umfasst, wobei die Teilchenabscheidekammer einen Teilcheneinlass zum Einführen von absorbierenden Teilchen in die Teilchenabscheidekammer umfasst,
**dadurch gekennzeichnet, dass** die Vorrichtung ein Stoßmittel umfasst, wobei das Stoßmittel eine starre Platte (10) umfasst, die innerhalb der Teilchenabscheidekammer drehbar befestigt ist, wobei das Stoßmittel so ausgelegt ist, dass es schwingt, um absorbierende Teilchen, die durch den Teilcheneinlass eingehen, vor der Abscheidung der Teilchen auf das Substrat gemäß einem Verteilungsprofil zu verteilen, wobei das Verteilungsprofil ein im Wesentlichen periodisches längsgerichtetes Profil umfasst, wobei das längsgerichtete Profil in einer einzelnen Periode und vorzugsweise für jede Periode umfasst:
o mindestens eine minimale, von null verschiedene Teilchendichte über im Wesentlichen die vollständige Periodenlänge;
o eine maximale Teilchendichte in einer ersten längsgerichteten Position, und
o eine Insultregion, die sich höchstens über einen 50%igen Teil der Periodenlänge erstreckt und mindestens 60% der absorbierenden Teilchen umfasst, die auf dem Substrat über die Periodenlänge verteilt sind.

5. Vorrichtung nach Anspruch 4, wobei das Stoßmittel eine keilförmige starre Platte umfasst.

6. Vorrichtung nach Anspruch 4 oder 5, wobei das Stoßmittel entfernbar in der Teilchenabscheidekammer befestigt ist.

7. Vorrichtung nach einem der Ansprüche 4 bis 6, umfassend ein Endlosträgermittel, vorzugsweise eine drehende Trommel, zum Befördern des Endlossubstrats in der Maschinenrichtung.

8. Vorrichtung nach einem der Ansprüche 4 bis 7, umfassend eine Flusenabscheidekammer mit einer oder mehreren Öffnungen zum Befördern eines Substrats entlang einer Maschinenrichtung durch die Flusenabscheidekammer, wobei die Flusenabscheidekammer vor oder nach der Teilchenabscheidekammer entlang der Maschinenrichtung, vorzugsweise vor der Teilchenabscheidekammer entlang der Maschinenrichtung, angeordnet ist, wobei die Flusenabscheidekammer einen Einlass für Flusen oder für eine Mischung aus Flusen und absorbierenden Teilchen umfasst, und optional einen Einlass für absorbierende Teilchen umfasst.

9. Vorrichtung zum Bereitstellen von absorbierendem Material, das Flusen und absorbierende Teilchen umfasst, auf einem Endlossubtrat, wobei die Vorrichtung umfasst:
o ein Endlosträgermittel zum Befördern des Endlossubstrats in einer Maschinenrichtung;
o eine Vorrichtung zum Verteilen von absorbierenden Teilchen nach Anspruch 4 bis 8, wobei die Teilchenabscheidekammer einen Teilcheneinlass zum Einführen von absorbierenden Teilchen oder einer Mischung aus absorbierenden Teilchen und Flusen in die Teilchenabscheidekammer umfasst;
**dadurch gekennzeichnet, dass** die Vorrichtung eine Flusenabscheidekammer mit einer oder mehreren Öffnungen zum Befördern des Substrats entlang einer Maschinenrichtung durch die Flusenabscheidekammer umfasst, wobei die Flusenabscheidekammer einen Einlass für Flusen umfasst, wobei die Flusenabscheidekammer vor oder nach der Teilchenabscheidekammer entlang der Maschinenrichtung angeordnet ist,
und dadurch, dass die Teilchenabscheidekammer ein Befestigungsmittel zum drehbaren Befestigen eines Stoßmittels innerhalb der Teilchenabscheidekammer umfasst.

10. Vorrichtung nach Anspruch 9, wobei die Flusenabscheidekammer vor der Teilchenabscheidekammer entlang der Maschinenrichtung angeordnet ist.

11. Vorrichtung nach einem der Ansprüche 9 oder 10, umfassend eine zusätzliche Flusenabscheidekammer mit einer oder mehreren Öffnungen zum Befördern des Substrats entlang einer Maschinenrichtung durch die zusätzliche Flusenabscheidekammer, wobei die zusätzliche Flusenabscheidekammer einen Einlass für Flusen umfasst, wobei die zusätzliche Flusenabscheidekammer nach der Teilchenabscheidekammer entlang der Maschinenrichtung angeordnet ist.

12. Verfahren zur Bereitstellung von absorbierendem Material, das Flusen und absorbierende Teilchen umfasst, auf einem Endlossubstrat mit einer Vorrichtung, die ein Endlosträgermittel, eine Teilchenabscheidekammer, eine Flusenabscheidekammer und optional eine zusätzliche Flusenabscheidekammer umfasst, wobei das Verfahren die folgenden Schritte umfasst:
(a) Befördern eines Endlossubstrats, vorzugsweise eines Vlieses, auf einem Endlosträgermittel entlang einer Maschinenrichtung durch eine Flusenabscheidekammer und anschließend durch eine Teilchenabscheidekammer und danach optional durch eine zusätzliche Flusenabscheidekammer;
(b) optionales Abscheiden von Flusen auf das Substrat in der ersten Abscheidekammer;
(c) Abscheiden von absorbierenden Teilchen auf das Substrat in der zweiten Abscheidekammer, wobei die absorbierenden Teilchen optional mit Flusen gemischt werden;
(d) optionales Abscheiden von Flusen auf das Substrat in der dritten Abscheidekammer,
wobei das Verfahren unter Verwendung einer Vorrichtung nach einem der Ansprüche 9 bis 11 durchgeführt wird.

## Revendications

1. Procédé de distribution de particules absorbantes à un substrat sans fin, de préférence un intissé, qui est acheminé le long d'une direction machine à travers une chambre de dépôt de particules, en déviant les particules absorbantes entrantes à l'aide d'un moyen d'impact oscillant dans ladite chambre de dépôt de particules avant le dépôt, afin de distribuer les particules au substrat selon un profil de distribution, dans lequel ledit moyen d'impact comprend une plaque rigide (10), qui est installée de manière pivotante dans la chambre de dépôt de particules autour d'un axe de pivotement,
dans lequel ledit moyen d'impact oscille selon un profil d'oscillation prédéfini de sorte que les particules absorbantes soient distribuées au substrat selon un profil de distribution comprenant un profil dans le sens de la longueur sensiblement périodique moyennant quoi, en une seule période, et de préférence pendant chaque période, ledit profil dans le sens de la longueur comprend :
- au moins une densité de particules minimale non nulle sur la quasi totalité de la période ;
- une densité de particules maximale à un premier emplacement dans le sens de la longueur, et
- une région de rejet qui s'étend sur tout au plus 50% de la longueur de période et qui comprend au moins 60% des particules absorbantes distribuées sur ledit substrat pendant la période.

2. Procédé selon la revendication 1, dans lequel les particules sont des particules de polymère super-absorbantes et/ou dans lequel le substrat est un intissé.

3. Procédé selon la revendication 1 ou 2, dans lequel ledit moyen d'impact oscille autour d'un axe de pivotement au-dessus du substrat, l'axe de pivotement étant orienté le long d'une direction transversale principalement horizontale.

4. Appareil de distribution de particules absorbantes à un substrat sans fin et éventuellement fibreux destiné à être utilisé dans un procédé selon l'une quelconque des revendications précédentes, l'appareil comprenant une chambre de dépôt de particules comprenant une ou plusieurs ouverture(s) destinée(s) à acheminer le substrat le long d'une direction machine à travers la chambre de dépôt de particules, ladite chambre de dépôt de particules comprenant une admission de particules destinée à introduire des particules absorbantes dans la chambre de dépôt de particules,
**caractérisé en ce que** l'appareil comprend un moyen d'impact, dans lequel ledit moyen d'impact comprend une plaque rigide (10), qui est installée de manière pivotante dans la chambre de dépôt de particules, le moyen d'impact étant configuré pour osciller afin de dévier les particules absorbantes qui pénètrent par le biais de l'admission de particules avant le dépôt des particules sur le substrat selon un profil de distribution, ledit profil de distribution comprenant un profil dans le sens de la longueur quasiment périodique, moyennant quoi, en une seule période, et de préférence pendant chaque période, ledit profil dans le sens de la longueur comprend :
- au moins une densité de particules minimale non nulle sur la quasi totalité de la période ;
- une densité de particules maximale à un premier emplacement dans le sens de la longueur, et
- une région de rejet qui s'étend sur tout au plus 50% de la longueur de période et qui comprend au moins 60% des particules absorbantes distribuées sur ledit substrat pendant la période.

5. Appareil selon la revendication 4, dans lequel ledit moyen d'impact comprend une plaque rigide en forme de cale.

6. Appareil selon la revendication 4 ou 5, dans lequel le moyen d'impact est installé de manière amovible dans la chambre de dépôt de particules.

7. Appareil selon l'une quelconque des revendications 4 à 6, comprenant un moyen d'acheminement sans fin, de préférence un tambour rotatif, destiné à acheminer ledit substrat sans fin dans la direction machine.

8. Appareil selon l'une quelconque des revendications 4 à 7, comprenant une chambre de dépôt de fibres comprenant une ou plusieurs ouverture(s) destinée(s) à acheminer le substrat le long d'une direction machine à travers la chambre de dépôt de fibres, ladite chambre de dépôt de fibres étant disposée avant ou après ladite chambre de dépôt de particules le long de la direction machine, de préférence avant ladite chambre de dépôt de particules le long de la direction machine, la chambre de dépôt de fibres comprenant une admission destinée aux fibres ou à un mélange de fibres et de particules absorbantes, et comprenant éventuellement une admission destinée aux particules absorbantes.

9. Appareil destiné à fournir une matière absorbante comprenant des fibres et des particules absorbantes à un substrat sans fin, ledit appareil comprenant :
- un moyen d'acheminement sans fin destiné à acheminer ledit substrat sans fin dans une direction machine ;
- un appareil destiné à distribuer des particules absorbantes selon les revendications 4 à 8, ladite chambre de dépôt de particules comprenant une admission de particules destinée à introduire des particules absorbantes ou un mélange de particules absorbantes et de fibres dans la chambre de dépôt de particules ;
**caractérisé en ce que** l'appareil comprend une chambre de dépôt de fibres comprenant une ou plusieurs ouverture(s) destinée(s) à acheminer le substrat le long d'une direction machine à travers la chambre de dépôt de fibres, la chambre de dépôt de fibres comprenant une admission destinée aux fibres, dans lequel ladite chambre de dépôt de fibres est disposée avant ou après ladite chambre de dépôt de particules le long de la direction machine,
et **en ce que** la chambre de dépôt de particules comprend un moyen de montage destiné à installer de manière pivotante le moyen d'impact dans ladite chambre de dépôt de particules.

10. Appareil selon la revendication 9, dans lequel ladite chambre de dépôt de fibres est disposée avant ladite chambre de dépôt de particules le long de la direction machine.

11. Appareil selon l'une quelconque des revendications 9 ou 10, comprenant une chambre de dépôt de fibres supplémentaire comprenant une ou plusieurs ouverture(s) destinée(s) à acheminer le substrat le long d'une direction machine à travers la chambre de dépôt de fibres supplémentaire, la chambre de dépôt de fibres supplémentaire comprenant une admission destinée aux fibres, dans lequel ladite chambre de dépôt de fibres supplémentaire est disposée après ladite chambre de dépôt de particules le long de la direction machine.

12. Procédé destiné à fournir une substance absorbante comprenant des fibres et des particules absorbantes à un substrat sans fin avec un appareil comprenant un moyen d'acheminement sans fin, une chambre de dépôt de particules, une chambre de dépôt de fibres et éventuellement une chambre de dépôt de fibres supplémentaire, le procédé comprenant les étapes consistant à :
(a) acheminer un substrat sans fin, de préférence un intissé, sur un moyen d'acheminement sans fin le long d'une direction machine à travers une chambre de dépôt de fibres puis à travers une chambre de dépôt de particules puis éventuellement à travers une chambre de dépôt de fibres supplémentaire ;
(b) déposer éventuellement des fibres sur ledit substrat dans ladite première chambre de dépôt ;
(c) déposer des particules absorbantes sur ledit substrat dans ladite seconde chambre de dépôt, lesdites particules absorbantes étant éventuellement mélangées avec des fibres ;
(d) déposer éventuellement des fibres sur ledit substrat dans ladite troisième chambre de dépôt de fibres,
ledit procédé étant exécuté à l'aide d'un appareil selon l'une quelconque des revendications 9 à 11.
